(19) Europäisches Patentamt
European Patent Office
Office européen des brevets

(11) **EP 1 371 697 A2**

(12) **EUROPEAN PATENT APPLICATION**

(43) Date of publication:
**17.12.2003 Bulletin 2003/51**

(51) Int Cl.⁷: **C09D 11/00**, C08J 3/07,
C08F 2/06, C08J 3/26

(21) Application number: **03253676.5**

(22) Date of filing: **11.06.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **14.06.2002 US 389043 P**
**30.09.2002 US 414599 P**

(71) Applicant: **ROHM AND HAAS COMPANY**
**Philadelphia, Pennsylvania 19106-2399 (US)**

(72) Inventors:
• **Fu, Zhenwen**
**Lansdale, Pennsylvania 19446 (US)**
• **Graziano, Louis Christopher**
**Doylestown, Pennsylvania 18901 (US)**

• **Lein, George Max**
**Chalfont, Pennsylvania 18914 (US)**
• **Hallden-Abberton, Michael Paul**
**Maple Glen, Pennsylvania 19002 (US)**
• **Lundquist, Eric Gustave**
**North Wales, Pennsylvania 19454 (US)**
• **Devonport, Wayne**
**Doylestown, Pennsylvania 18901 (US)**

(74) Representative: **Kent, Venetia Katherine**
**Rohm and Haas (UK) Ltd**
**European Operations Patent Dept.**
**Lennig House**
**2 Mason's Avenue**
**Croydon, CR9 3NB (GB)**

(54) **Polymeric binders for inkjet inks**

(57)     An inkjet ink binder including polymeric nanoparticles ("PNPs") having a mean diameter in the range of 1 to 50 nanometers, wherein the PNPs include as polymerized units 1-20%, by weight based on dry polymer weight, of a curable composition. The reaction of the curable composition can be initiated thermally, chemically or via actinic radiation. An inkjet ink composition comprising a liquid medium, a colorant and the inkjet binder and a method for improving the durability of inkjet ink printed on a substrate are also provided.

**Description**

This invention relates to polymeric binders for inkjet inks. In particular, this invention relates to an inkjet ink binder including polymeric nanoparticles ("PNPs") having a mean diameter in the range of 1 to 50 nanometers, wherein the PNPs include as polymerized units 1-20%, by weight based on dry polymer weight, of a curable composition. The reaction of the curable composition can be initiated thermally, chemically or via actinic radiation. This invention is also related to an inkjet ink composition comprising a liquid medium, a colorant and the inkjet binder and also a method for improving the durability of inkjet ink printed on a substrate.

Ink jet printing is a well established technique for applying an ink to a substrate to form an image, in which there is no physical contact between the functional part of the printer from which the ink is applied and the substrate onto which the ink is deposited. The ink is applied in the form of micro-droplets, which are projected by well known means through small nozzles in the print head onto the substrate.

O.Y. Tian and W.C. Tincher, "Pigmented Latex System for Ink Jet Printing on Textile," Proceedings of IS&T NIP 15: 1999 International Conference on Digital Printing Technologies, 196-199 (1999) discloses small particle size polymer latex systems in ink jet printing on textile substrates, including "curing" to evaporate water and form an integrated polymer film.

International patent application WO 99/50365 describes the use of small particle size (<60 nm) acrylic polymers dispersed using steric stabilizers such as polyethyleneglycol, to provide ink jet inks with improved stability. The compositions disclosed in WO 99/50365 do not require the use of highly crosslinked particles, nor is the value of using a highly crosslinked particle recognized. In addition, the value of having a curable functionality in the small particle size dispersion is not anticipated.

International patent application WO 01/88046 discloses the use of polymer systems that when formulated with hydrophobic dyes and volatile organics are capable of forming nanoparticle size materials. This aplication teaches the use of uncrosslinked polymers that do not exist as nanoparticles in the wet state, relying on the evaporation of volatile organics from the jetting fluid after application to a substrate to achieve nanoparticle formation.

International patent application WO 01/90226 discloses the preparation of polymeric nanoparticles through crosslinking of already prepared polymers that have been swollen in solvent prior to crosslinking. This application requires preparation of polymers prior to the swelling and crosslinking step, and therefore is limited in composition and crosslinking chemistry. WO 01/90226 does not anticipate thermal or radiation curing of the binder composition after it is applied.

The problem addressed by the present invention is to provide a binder capable of cross-linking, for use in ink jet inks exhibiting improved durability, such as wash-fastness, to the print on a substrate, such as a textile. The binder also imparts properties to the ink jet ink such as good solvent stability, jettability, wettability, viscosity stability to monomer addition, surface tension, optical density and image quality.

The present invention provides a binder composition comprising polymeric nanoparticles ("PNPs") having a mean diameter in the range of 1 to 50 nanometers, the PNPs comprising as polymerized units 1-20%, by weight based on dry polymer weight, of one or more curable compositions which are unreactive at ambient conditions and are capable of being intiated thermally, chemically or via actinic radiation. The present invention further provides a method for improving the durability of inkjet ink printed on a substrate comprising: (a) forming an inkjet ink composition comprising a liquid medium, a colorant and a binder composition comprising polymeric nanoparticles ("PNPs") having a mean diameter in the range of 1 to 50 nanometers, the PNPs comprising as polymerized units 1-20%, by weight based on dry polymer weight, of one or more curable compositions which are unreactive at ambient conditions and are capable of being intiated thermally, chemically or via actinic radiation; (b) printing the ink on a substrate; and (c) curing the ink by applying thermal or radiation energy.

Surprisingly, the addition of a curable composition to the PNP that can be initiated thermally, chemically or via actinic radiation, provides binders for use in ink jet inks that exhibit improved properties, including on textile substrates.

The aqueous composition of the present invention includes an aqueous dispersion of polymeric particles having a mean diameter in the range of from 1 to 50 nanometers (nm), the particles including, as polymerized units, at least one multiethylenically unsaturated monomer and at least one ethylenically unsaturated water soluble monomer. As used herein, the term "dispersion" refers to a physical state of matter that includes at least two distinct phases wherein a first phase is distributed in a second phase, the second phase being a continuous medium. By "aqueous" herein is meant a medium that is from 50 to 100 weight % water, based on the weight of the aqueous medium.

The polymeric particles, referred to herein as polymeric nanoparticles ("PNPs"), are addition polymers, which contain, as polymerized units, at least one multiethylenically unsaturated monomer and at least one ethylenically unsaturated water soluble monomer. Suitable multiethylenically unsaturated monomers useful in the present invention include di-, tri-, tetra-, or higher multifunctional ethylenically unsaturated monomers, such as, for example, divinyl benzene, trivinylbenzene, divinyltoluene, divinylpyridine, divinylnaphthalene divinylxylene, ethyleneglycol di(meth)acrylate, trimethylolpropane tri(meth)acrylate, diethyleneglycol divinyl ether, trivinylcyclohexane, allyl (meth)acrylate, diethyleneglycol di(meth)acrylate, propyleneglycol di(meth)acrylate, 2,2-dimethylpropane-1,3-di(meth)acrylate, 1,3-butylene glycol di(meth)acrylate, 1,4-butanediol di(meth)acrylate, 1,6-hexanediol di(meth)acrylate, tripropylene glycol di(meth)acrylate, triethylene glycol di(meth)acrylate, tetraethylene glycol di(meth)acrylate, polyethylene glycol di(meth)acrylates, such as polyethylene glycol 200 di(meth)acrylate and polyethylene glycol 600 di(meth)acrylate, ethoxylated bisphenol A di(meth)acrylate, poly(butanediol) di(meth)acrylate, pentaerythritol tri(meth)acrylate, trimethylolpropane triethoxy tri(meth)acrylate, glyceryl propoxy tri(meth)acrylate, pentaerythritol tetra(meth)acrylate, dipentaerythritol monohydroxypenta(meth)acrylate, divinyl silane, trivinyl silane, dimethyl divinyl silane, divinyl methyl silane, methyl trivinyl silane, diphenyl divinyl silane, divinyl phenyl silane, trivinyl phenyl silane, divinyl methyl phenyl silane, tetravinyl silane, dimethyl vinyl disiloxane, poly(methyl vinyl siloxane), poly(vinyl hydro siloxane), poly(phenyl vinyl siloxane), and mixtures thereof. The term "(meth)acrylic" includes both acrylic and methacrylic and the term "(meth) acrylate" includes both acrylate and methacrylate. Likewise, the term "(meth)acrylamide" refers to both acrylamide and methacrylamide. "Alkyl" includes straight chain, branched and cyclic alkyl groups.

Typically, the PNPs contain at least 1 % by weight based on the weight of the PNPs, of at least one polymerized multiethylenically unsaturated monomer. Up to and including 99.5 weight % polymerized multiethylenically unsaturated monomer, based on the weight of the PNPs, is effectively used in the particles of the present invention. It is preferred that the amount of polymerized multiethylenically unsaturated monomer is from 1% to 80%, more preferably from 1% to 60%, most preferably from 1% to 25%, by weight based on the weight of the PNPs.

The PNPs further contain, as polymerized units, at least one water soluble monomer. By "water soluble monomer" herein is meant a monomer having a solubility in water of at least 7 weight %, preferably at least 9 weight %, and most preferably as least 12 weight %, at a temperature of 25 °C. Data for the water solubility of monomers is found, for example, in "Polymer Handbook" (Second Edition, J. Brandrup, E.H. Immergut, Editors, John Wiley & Sons, New York) and "Merck Index" (Eleventh Edition, Merck & Co, Inc., Rahway, New Jersey). Examples of water soluble monomers include ethylenically unsaturated ionic monomers and ethylenically unsaturated water soluble nonionic monomers. Typically, the amount of the polymerized water soluble monomer is at least 0.5 weight %, based on the weight of the PNPs. Up to and including 99 weight % polymerized water soluble monomer, based on the weight of the PNPs, can be effectively used in the particles of the present invention.

Ethylenically unsaturated ionic monomer, referred to herein as "ionic monomer" is a monomer that is capable of bearing an ionic charge in the aqueous medium in which the PNPs are dispersed. Suitable ionic monomers include, for example, acid-containing monomers, base-containing monomers, amphoteric monomers; quaternized nitrogen-containing monomers, and other monomers that can be subsequently formed into ionic monomers, such as monomers which can be neutralized by an acid-base reaction to form an ionic monomer. Suitable acid groups include carboxylic acid groups and strong acid groups, such as phosphorus containing acids and sulfur containing acids. Suitable base groups include amines. It is preferred that the amount of polymerized ionic monomer based on the weight of the PNPs is in the range from 0.5 to 99 weight %, more preferably in the range of from 1 to 50 weight %, even more preferably from 2 to 40 weight %, and most preferably from 3 to 25 weight %.

Suitable carboxylic acid-containing monomers include carboxylic acid monomers, such as (meth)acrylic acid, acryloxypropionic acid, and crotonic acid; dicarboxylic acid monomers, such as itaconic acid, maleic acid, fumaric acid, and citraconic acid; and monomers which are half esters of dicarboxylic acids, such as monomers containing one carboxylic acid functionality and one $C_{1-6}$ ester. Preferred are acrylic acid and methacrylic acid. Suitable strong acid monomers include sulfur acid monomers, such as 2-acrylamido-2-methyl propane sulfonic acid, styrene sulfonic acid, vinyl sulfonic acid, sulfoethyl (meth)acrylate, sulfopropyl (meth)acrylate, 2-acrylamido-2-methyl propane sulfinic acid, styrene sulfinic acid, and vinyl sulfinic acid; and phosphorus acid monomers, such as 2-phosphoethyl (meth)acrylate, vinyl phosphoric acid, and vinyl phosphinic acid. Other acid monomers include terminally unsaturated acid containing macromonomers as disclosed in U.S. Patent No. 5,710,227. Phosphorus acid monomers are desirable as they can provide improved adhesion to certain substrates (e.g., metal).

Suitable base-containing monomers include monomers having amine functionality, which includes N,N-dimethylaminoethyl (meth)acrylate, N,N-diethylaminoethyl (meth)acrylate, N-t-butylaminoethyl (meth)acrylate, N,N-dimethylaminopropyl (meth)acrylamide, p-aminostyrene, N,N-cyclohexylallylamine, allylamine, diallylamine, dimethylallylamine, N-ethyldimethylallylamine, crotyl amines, and N-ethylmethallylamine; monomers having pyridine functionality, which includes 2-vinylpyridine and 4-vinylpyridine; monomers having piperidine functionality, such as vinylpiperidines; and monomers having imidazole functionality, which includes vinyl imidazole. Other suitable base-containing monomers include oxazolidinylethyl (meth)acrylate, vinylbenzylamines, vinylphenylamines, substituted diallylamines, 2-morpholinoethyl (meth)acrylate, methacrylamidopropyl trimethyl ammonium chloride, diallyl dimethyl ammonium chloride, 2-trimethyl ammonium ethyl methacrylic chloride, and the like.

Suitable amphoteric monomers include N-vinylimidazolium sulfonate inner salts and N,N-Dimethyl-N-(3-methacrylamidopropyl)-N-(3-sulfopropyl) ammonium betaine.

Suitable functional monomers, in which the functionality is subsequently formed into an acid or base include monomers containing: an epoxide functionality, such as glycidyl (meth)acrylate and allyl glycidyl ether; an anhydride, such as maleic anhydride; an ester such as methyl acrylate; and a halide. Suitable halide-containing functional monomers include vinylaromatic halides and halo-alkyl(meth)acrylates. Suitable vinylaromatic halides include vinylbenzyl chloride and vinylbenzyl bromide. Other suitable functional monomers include allyl chloride, allyl bromide, and (meth) acrylic acid chloride. Suitable halo-alkyl(meth)acrylates include chloromethyl (meth)acrylate. Suitable functional monomers, in which the functionality is subsequently forming into a nonionic water soluble group include vinyl acetate. Hydrolysis of the polymerized vinyl acetate provides hydroxyl groups to the PNPs.

Multiethylenically unsaturated monomers that are also water soluble monomers are alternatively used to prepare the PNPs. In such embodiments, these monomers are classified for the purposes of the present invention as both a multiethylenically unsaturated monomer and a water soluble monomer. An example of a water soluble, multiethylenically unsaturated monomer is phosphodi(ethyl methacrylate).

Ethylenically unsaturated water soluble nonionic monomers are referred to herein as "water soluble nonionic monomers". Examples of water soluble nonionic monomers include hydroxyalkyl (meth)acrylates such as hydroxyethyl (meth)acrylate and hydroxypropyl (meth)acrylate; poly(alkylene oxide) esters of (meth)acrylic acid such as poly(ethylene oxide)$_{20}$ methacrylate and poly(propylene oxide)$_{150}$ acrylate; acrylamide; and methacrylamide. It is preferred that the amount of polymerized water soluble nonionic monomer based on the weight of the PNPs is in the range from 0.5 to 99 weight %, more preferably in the range of from 20 to 90 weight %, even more preferably from 30 to 80 weight %, and most preferably from 40 to 70 weight %. When the PNPs include, as polymerized units, ionic monomer and nonionic water soluble monomer, lower levels of polymerized nonionic water soluble monomer are preferred.

The PNPs optionally contain, as polymerized units, one or more third monomers that are not multiethylenically unsaturated monomers and are not water soluble monomers. Suitable third monomers include $C_1$-$C_{24}$ alkyl (meth) acrylates, such as methyl (meth)acrylate, ethyl (meth)acrylate, propyl (meth)acrylate, butyl (meth)acrylate, isobutyl (meth)acrylate, hexyl (meth)acrylate, cyclohexyl (meth)acrylate, 2-ethylhexyl (meth)acrylate, octyl (meth)acrylate, decyl (meth)acrylate, dodecyl (meth)acrylate, pentadecyl (meth)acrylate, hexadecyl (meth)acrylate, octadecyl (meth)acrylate, and nonadecyl (meth)acrylate, and mixtures thereof. Other suitable third monomers include vinyl acetate; vinyl versatate; diisobutylene; ureido containing monomers such as N-(ethyleneureidoethyl)-4-pentenamide, N-(ethylenethioureido-ethyl)-10-undecenamide, butyl ethyleneureido-ethyl fumarate, methyl ethyleneureido-ethyl fumarate, benzyl N-(ethyleneureido-ethyl) fumarate, and benzyl N-(ethyleneureido-ethyl) maleamate; vinylaromatic monomers, such as styrene, α-methylstyrene, vinyltoluene, p-methylstyrene, ethylvinylbenzene, vinylnaphthalene, vinylxylenes, and nonylphenoxy propenyl polyethoxylated alcohol. The vinylaromatic monomers also include their corresponding substituted counterparts, such as halogenated derivatives, i.e., containing one or more halogen groups, such as fluorine, chlorine or bromine; and nitro, cyano, ($C_1$-$C_{10}$)alkoxy, halo($C_1$-$C_{10}$)alkyl, ($C_1$-$C_{10}$)alkoxy, carboxy, and the like.

The PNPs have a mean diameter in the range of from 1 to 50 nm, preferably in the range of from 1 to 40 nm, more preferably from 1 to 30 nm, even more preferably from 1 to 25 nm, even further preferably from 1 to 20 nm, and most preferably from 1 to 10 nm. It is further typical that the PNPs have a mean particle diameter of at least 1.5 nm, preferably at least 2 nm. One method of determining the particle sizes (mean particle diameter) of the PNPs is by using standard dynamic light scattering techniques, wherein the correlation functions are converted to hydrodynamic sizes using LaPlace inversion methods, such as CONTIN.

Typically, PNPs including as polymerized units, less than 10 weight % multiethylenically unsaturated monomer, have a glass transition temperature from -90 °C to 170 °C for the composition in the absence of the polymerized multiethylenically unsaturated monomer, as determined by a modulated differential scanning calorimetry measurement. PNPs containing as polymerized units, at least 50 weight % multiethylenically unsaturated monomer are considered to have glass transition temperatures of at least 50°C.

The PNPs of the present invention typically have an "apparent weight average molecular weight" in the range of 5,000 to 1,000,000, preferably in the range of 10,000 to 500,000 and more preferably in the range of 15,000 to 100,000. As used herein, "apparent weight average molecular weight" reflects the size of the PNP particles using standard gel permeation chromatography methods, e.g., using THF solvent at 40 °C, 3 PIgel™ Columns (Polymer Labs, Amherst, MA), 100 Angstrom (10 nm), $10^3$ Angstroms (100 nm), $10^4$ Angstroms (1 micron), 30 cm long, 7.8 mm ID, 1 milliliter per minute, 100 microliter injection volume, calibrated to narrow polystyrene standards using Polymer Labs CALIBRE ™ software.

The PNPs are optionally characterized as having suitable hydrophilicities that allow the PNPs to be dispersed into an aqueous medium. One method to characterize the hydrophilicity of the PNPs is to calculate the Hansch parameter. The Hansch parameter is calculated using a group contribution method. The monomer units forming the polymer are assigned a hydrophobicity contribution and the relative hydrophobicity of the polymer is calculated based on the weight average of the monomers in the polymer. Hansch and Fujita, *J. Amer. Chem. Soc.,* 86, 1616-1626 (1964); H. Kubinyi, *Methods and Principles of Medicinal Chemistry,* Volume 1, R. Mannhold et al., Eds., VCH, Weinheim (1993); C. Hansch and A. Leo, *Substituent Constants for Correlation Analysis in Chemistry and Biology,* Wiley, New York (1979); and C. Hansch, P. Maloney, T. Fujita, and R. Muir, *Nature,* 194. 178-180(1962).

Values of the hydrophobicity contributions for several monomers are listed in Table 1.

Table 1

| Monomer | Hydrophobicity Contribution |
|---|---|
| ethyl acrylate | 2.11 |
| butyl acrylate | 3.19 |
| 2-ethyl hexylacrylate | 5.22 |
| styrene | 4.29 |
| methyl methacrylate | 1.89 |
| ethyl methacrylate | 2.43 |
| butyl methacrylate | 3.51 |
| isobornyl methacrylate | 5.0 |
| butadiene | 4.0 |
| acrylic acid | -2.52 |
| methacrylic acid | -2.2 |
| maleic anhydride | -3.5 |

Preferred PNPs have a Hansch parameter in the range of from -2.5 to 4, alternatively from -1 to 3.

The PNPs optionally contain other functional groups, which are provided by the polymerization of monomers containing those groups or precursor groups thereof. Functional groups are optionally attached to the PNPs by reacting the ionic group of the PNP with a suitable compound. For example, PNPs containing carboxylic acid groups are modified to contain pendant hydrophilic groups by reacting carboxylic acid groups with a suitable alcohol, such as a capped polyalkylene oxide. Alternatively, functional groups are affixed to the PNPs through non-radical reactions resulting in the formation of ionic or covalent bonds between a modifying compound containing the groups and complementary reactable groups covalently bound to the PNP as taught in U.S. Patent No. 5,270,380.

The complementary reactable groups in the PNP and modifying compound provide ionic or covalent bonding. Complementary ionic bonding includes acid-base interaction and ion pair bonding of negatively and positively charged atoms. Covalent bonding by complementary reactable groups includes, for example: (a) acetoacetate-aldehyde; (b) acetoacetate-amine; c) amine-aldehyde; (d) amine-anhydride; (e) amine-isocyanate; (f) amine-epoxy; (g) aldehyde-hydrazide; (i) acid-epoxy; (j) acid-carbodiimide; (k) acid-chloro methyl ester; (j) acid-chloro methyl amine; (m) acid-anhydride; (n) acid-aziridine; (o) epoxy-mercaptan; and (p) isocyanate-alcohol. The first or second reactable group in each pair is present either in the PNP or, alternatively, in the modifying compound.

A suitable method to prepare the aqueous composition containing the PNPs dispersed in an aqueous medium includes the steps of preparing a nonaqueous PNP dispersion containing the PNPs dispersed in at least one solvent; and combining the nonaqueous PNP dispersion with an aqueous medium. By "nonaqueous" herein is meant a medium that contains from zero to less than 50 weight % water, based on the weight of the nonaqueous medium. Aqueous compositions containing PNPs that include, as polymerized units, ionic monomers, are optionally partially or completely neutralized prior to, during, or after combining with the aqueous medium.

A suitable polymerization process to prepare the nonaqueous PNP dispersion is free radical solution polymerization of at least one multiethylenically unsaturated monomer, at least one water soluble monomer, and optionally, at least one third monomer. By "solution polymerization" herein is meant free radical addition polymerization in a suitable solvent for the polymer. By "suitable solvent for the polymer" herein is meant that linear random (co)-polymers having substantially similar polymerized monomer units to the PNPs, are soluble in the solvent. Another method for selecting a suitable solvent or mixture of solvents is on the basis of using solubility parameter analysis. According to such methods, the suitability of the solvent is determined by substantially matching the solubility parameters of the PNP and of the solvent, such as the Van Krevelen parameters of delta d, delta p, delta h and delta v. See, for example, Van Krevelen et al., Properties of Polymers. Their Estimation and Correlation with Chemical Structure, Elsevier Scientific Publishing Co., 1976; Olabisi et al., Polymer-Polymer Miscibility, Academic Press, NY, 1979; Coleman et al., Specific Interactions and the Miscibility of Polymer Blends, Technomic, 1991; and A. F. M. Barton, CRC Handbook of Solubility Parameters and Other Cohesion Parameters, 2nd Ed., CRC Press, 1991. Delta d is a measure of dispersive interactions, delta p is a measure of polar interactions, delta h is a measure of hydrogen bonding interactions, and delta v is a measure of both dispersive and polar interactions. Such solubility parameters are alternatively calculated, such as by the group contribution method, or determined experimentally, as is known in the art. A preferred solvent has a delta v parameter within 5 (joule per cubic centimeter)$^{1/2}$, preferably within 1 (joule per cubic centimeter)$^{1/2}$ of the polymer delta v parameter. Suitable solvents for the polymerization include organic solvents, such as hydrocarbons; alkanes; halohydrocarbons; chlorinated, fluorinated, and brominated hydrocarbons; aromatic hydrocarbons; ethers; ketones; esters; alcohols; and mixtures thereof. Particularly suitable solvents, depending on the composition of the PNP, include dodecane, mesitylene, xylenes, diphenyl ether, gamma-butyrolactone, ethyl acetate, ethyl lactate, propyleneglycol monomethyl ether acetate, caprolactone, 2-heptanone, methylisobutyl ketone, acetone, methyl ethyl ketone, diisobutylketone, propyleneglycol monomethyl ether, alkyl-alcohols, such as isopropanol, decanol, and t-butanol; and supercritical carbon dioxide.

The nonaqueous PNP dispersion is prepared by first charging a solvent, or alternatively, a mixture of solvent and some portion of the monomers, to a reaction vessel. The monomer charge is typically composed of monomers, an initiator, and a chain transfer agent. Typically, initiation temperatures are in the range of from 55 °C to 125 °C, although lower or higher initiator temperatures are possible using suitable low temperature or high temperature initiators known in the art. After the heel charge has reached a temperature sufficient to initiate polymerization, the monomer charge or balance of the monomer charge is added to the reaction vessel. The monomer charge time period is typically in the range of from 15 minutes to 4 hours, although both shorter and longer time periods are envisioned. During the monomer charge, the reaction temperature is typically kept constant, although it is possible to vary the reaction temperature. After completing the monomer mixture addition, additional initiator in solvent can be charged to the reaction and/or the reaction mixture may be held for a time.

Control of PNP particle size and distribution is achieved by one or more of such methods as choice of solvent, choice of initiator, total solids level, initiator level, type and amount of multi-functional monomer, type and amount of ionic monomer, type and amount of chain transfer agent, and reaction conditions.

Initiators useful in the free radical polymerization of the present invention include, for example, one or more of: peroxyesters, alkylhydroperoxides, dialkylperoxides, azoinitiators, persulfates, redox initiators and the like. The amount of the free radical initiator used is typically from 0.05 to 10% by weight, based on the weight of total monomer. Chain transfer reagents are optionally used to control the extent of polymerization of the PNPs useful in the present invention. Suitable chain transfer agents include, for example: alkyl mercaptans, such as dodecyl mercaptan; aromatic hydrocarbons with activated hydrogens, such as toluene; and alkyl halides, such as bromotrichloroethane.

In one method of preparing the aqueous composition of the present invention, at least a portion of the polymerized ionic monomer units of the PNPs are neutralized with at least one neutralizing agent to form an at least partially neutralized nonaqueous PNP dispersion. The polymerized ionic monomer units of the PNPs can be neutralized in a variety of ways. When the polymerized ionic monomer units are acidic, the neutralizing agent is typically a base. Likewise, when the polymerized ionic monomer units are basic, the neutralizing agent is typically an acid. Suitable bases include inorganic and organic bases. Suitable inorganic bases include the full range of the hydroxide, carbonate, bicarbonate, and acetate bases of alkali or alkaline metals. Suitable organic bases include ammonia, primary/secondary/tertiary amines, diamines, and triamines. Preferred basic neutralizing agents include sodium hydroxide, and ammonium hydroxide. Suitable acids include carboxylic acids, such as acetic acid; dicarboxylic acids; (di)carboxylic/hydroxyl acids; aromatic acids, such as benzoic acid; and a variety of other acids, such as boric, carbonic, citric, iodic, nitrous, nitric, periodic, phosphoric, phosphorous, sulfuric, sulfurous, and hydrochloric acid. None of the foregoing categories of bases and acids, are deemed to be limiting.

The amount of neutralizing agent required to neutralize the nonaqueous PNP dispersion is typically determined on a molar basis of neutralizing agent to polymerized ionic monomer units of the PNPs. Without being bound to a particular theory, the amount of polymerized ionic monomer units (i.e., level of charge) needed to stabilize the PNPs (i.e., maintain particle size during conversion from nonaqueous to aqueous medium) will vary as PNP composition and properties are varied. It is believed that the PNP hydrophobicity, Tg, crosslinking level, and type of counter-ion from the neutralizing agent are important variables. For providing stable aqueous PNP dispersions (i.e., wherein flocculation of the PNPs is minimized), the polymerized ionic monomer units are preferably at least 20%, more preferably at least 50%, even more preferably at least 80%, and most preferably at least 90% neutralized.

Neutralizing the PNPs is alternatively carried out in a variety of ways. In one method, the nonaqueous PNP dispersion is added to a solution containing the neutralizing agent while stirring. Preferably, the neutralizing agent is added as an aqueous solution over time while stirring the nonaqueous PNP dispersion to provide an at least partially neutralized nonaqueous PNP dispersion.

In one method of preparing the aqueous composition containing dispersed PNPs, the at least partially neutralized nonaqueous PNP dispersion is combined with an aqueous medium. The aqueous medium optionally contains the neutralizing agent(s) for neutralizing the PNPs, in which case the nonaqueous PNP dispersion is capable of being simultaneously neutralized and combined with an aqueous medium. The aqueous medium optionally contains surfactants, which are capable of altering the stability of the PNPs, or of altering other properties of the resulting aqueous PNP dispersion, such as its surface tension.

The sequence of admixing the partially neutralized nonaqueous PNP dispersion and the aqueous medium is not critical. Various methods and equipment, which are suitable for mixing are described in *The Chemical Engineer's Handbook, 5th Edition,* Perry and Chilton, Eds., McGraw-Hill, Ch. 21, 1973. Typically, the aqueous medium is continuously stirred while adding the partially neutralized nonaqueous PNP dispersion to it in order to ensure that the solvent is intimately mixed with the aqueous medium, which minimizes flocculation of the PNPs.

Suitable weight percentages of the PNPs in the aqueous composition, based on total weight of the aqueous composition, are typically from 1 to 90 weight %, more typically from 2 to 75 weight %, even more typically from 4 to 65 weight %, further more typically from 8 to 55 weight %, and most typically from 10 to 45 weight %.

While the preparation of the aqueous composition of the present invention does not require the use of surfactants, and it is typical that the nonaqueous PNP dispersions are substantially free of surfactants, surfactants are optionally included. When present, the amount of surfactants is typically less than 3 weight percent, more typically less than 2 weight percent, even more typically less than 1 weight percent, further typically less than 0.5 weight percent, and even further typically less than 0.2 weight percent, based on total weight of the PNPs.

The aqueous composition is optionally treated to remove at least a portion of the solvent and optionally water, to increase the solids content of the PNPs. Suitable methods to concentrate the PNPs include distillation processes, such as forming azeotropes of water and a suitable solvent; evaporation of solvent or water; drying the aqueous composition by freeze drying or spray drying; solvent extraction techniques; and ultrafiltration techniques. Preferably at least 25 weight %, more preferably at least 50 weight %, even more preferably at least 75 weight %, and most preferably 100 weight % of the solvent is exchanged with water. Removal of the solvent is preferably carried out under conditions that minimize destabilization (i.e., flocculation) of the PNPs.

In an alternative method, the aqueous composition of this invention is prepared by a method including the steps of preparing a nonaqueous PNP dispersion containing the PNPs dispersed in at least one solvent that is both a suitable solvent for the PNPs and is compatible or miscible in water; and combining the nonaqueous PNP dispersion with an aqueous medium. Examples of such suitable solvents for acrylic-containing PNPs, which are also compatible or miscible with water, include isopropanol and ether alcohols (e.g., monobutyl ether of ethylene glycol and monoethyl ether of diethylene glycol). In this method, the PNPs do not require the addition of neutralizing agents to impart particle stability when combined with water.

Alternate embodiments of the aqueous compositions of the present invention have a wide range of PNP content. Typically, the PNP weight fractions range from 0.1 to 99 weight %, more typically from 1 to 90 weight %, even more typically from 2 to 75 weight %, further typically from 5 to 50 weight %, and most typically 10 to 40 weight %, based on the weight of the aqueous composition.

In combination with the above compositions or monomers used for preparing PNP compositions, the curable PNP compositions of this invention include, as polymerized units, 1-20%, preferably 3-10%, more preferably 4-8%, by weight based on dry polymer weight, of a first monomer selected from the group consisting of methylol acrylamide, methylol methacrylamide, methyl acrylamidoglycolate methyl ether, acrylamidoglycolic acid and mixtures thereof.

The curable compositions or monomers may include chemical/thermal curing combinations, one or both of which may be present in the PNP. In some formulations, one composition will be in the PNP and the other composition will be post added during preparation of an ink formulation, thereby providing the curing mechanism. The curable composition is selected from the group consisting of (a) methylol acrylamide, methylol methacrylamide, methyl acrylamidoglycolate methyl ether, acrylamidoglycolic acid; (b) a polyacid comprising at least two carboxylic acid groups, anhydride groups, or salts thereof, wherein said carboxylic acid groups, anhydride groups, or salts thereof are neutralized to an extent of less than about 35% with a fixed base; (c) a polyol comprising at least two hydroxyl groups or hydroxylamine selected from the group consisting of diisopropanolamine, 2-(2-aminoethylamino)- ethanol, triethanolamine, tris(hydroxymethyl)aminomethane, and diethanolamine; (d) a copolymerized ethylenically-unsaturated monomer having a pendant group selected from the group consisting of acetoacetate, acetoacetamide, cyanoacetate, cyanoacetamide, an alkyl polyglycoside and glycidyl(meth)acrylate; (e) a covalently bonded compound having at least one unreacted isocyanate functional group; (f) a copolymerized ethylenically-unsaturated monomer consisting of at least one sulfur- or phosphorous-containing acid group having an acid number of from 5 to 100, including 2-(meth) acrylamido-2-methyl-1- propanesulfonic acid, 3-sulfopropyl (meth)acrylate, 2-sulfoethyl (meth)acrylate, and 2-phosphoethyl (meth)acrylate; (g) an aliphatic polycarbodiimide; and (h) mixtures therof.

The curable compositions or monomers may include chemical/radiation curing combinations, one or both of which may be present in the PNP. In some formulations, one composition will be in the PNP and the other composition will be post added during preparation of an ink formulation, thereby providing the curing mechanism. The curable composition may include one or more of the compositions described above and glycidylmethacrylate.

The curable compositions or monomers may include a functional group that can undergo Diels Alder reactions upon curing with thermal or actinic radiation. In some formulations, one composition will be in the PNP and the other composition will be post added during preparation of an ink formulation, thereby providing the curing mechanism. The curable composition may include one or more of ethylene glycoldicyclopentenyl ether acrylate, ethylene glycol dicyclopentadienyl ether methacrylate and furfuryl methacrylate.

The glass transition temperature ("Tg") of the polymeric particles is typically from -50 °C to 150 °C, the monomers and amounts of the monomers selected to achieve the desired polymer Tg range being well known in the art. Typical Tg values for hollow micro-spheres are greater than 70 °C. "Glass transition temperature" or "$T_g$" as used herein, means the temperature at or above which a glassy polymer will undergo segmental motion of the polymer chain. Glass transition temperatures of a polymer can be estimated by the Fox equation [Bulletin of the American Physical Society 1, 3, page 123 (1956)] as follows:

$$\frac{1}{T_g} = \frac{w_1}{T_{g(1)}} + \frac{w_2}{T_{g(2)}}$$

For a copolymer of monomers $M_1$ and $M_2$, $w_1$ and $w_2$ refer to the weight fraction of the two co-monomers, and $T_{g(1)}$ and $T_{g(2)}$ refer to the glass transition temperatures of the two corresponding homopolymers in degrees Kelvin. For polymers containing three or more monomers, additional terms are added ($w_n/T_{g(n)}$). The $T_g$ of a polymer can also be measured by various techniques including, for example, differential scanning calorimetry ("DSC"). The particular values of $T_g$ reported herein are calculated based on the Fox equation. The glass transition temperatures of homopolymers may be found, for example, in "Polymer Handbook", edited by J. Brandrup and E.H. Immergut, Interscience Publishers.

Inkjet ink compositions include a liquid medium, a colorant and the crosslinked PNP of the present invention. The liquid medium is typically predominantly water, preferably deionized water. The inkjet ink composition includes a colorant which can be a pigment or dye. The pigment may be an organic pigment or an inorganic pigment. "Organic pigment" means a pigment which is predominantly an organic compound or mixture of organic compounds, including carbon black.

Suitable organic pigments include, for example, surface modified and unmodified, anthroquinones, phthalocyanine blues, phthalocyanine greens, diazos, monoazos, heterocyclic yellows, pyranthrones, quinacridone pigments, dioxazine pigments, indigo, thioindigo pigments, perynone pigments, perylene pigments, isoindolene, polymer particles having at least one void, and the like. Carbon black is the generic name for small particle size carbon particles formed in the gas phase by the thermal decomposition of hydrocarbons and includes, for example, materials known in the art as furnace black, lampblack, channel black, acetylene black. Carbon black additionally encompasses treated, modified, and oxidized cabon black. Suitable inorganic pigments include titanium dioxide, iron oxide, and other metal powders. Generally, the amount of pigment(s) used is less than 20%, preferably 3-8%, more preferably 2-6% by weight based on the total weight of the ink. The ink composition of the present invention preferably includes the emulsion polymer at a level of 0.1 to 25 %, more preferably 1 to 20%, by weight based on the total weight of the ink composition. The ink composition may also include water miscible or soluble materials such as polymers other than the curable PNPs of this invention, humectants, dispersants, penetrants, chelating agents, co-solvents, defoamers, buffers, biocides, fungicides, viscosity modifiers, bactericides, surfactants, anti-curling agents, anti-bleed agents and surface tension modifiers, all as is known in the art. Useful humectants include ethylene glycol, 1,3-propanediol, 1,4-butanediol, 1,4-cyclohexanedimethanol, 1,5-pentanediol, 1,6-hexanediol, 1,8-octanediol, 1,2-propanediol, 1,2-butanediol, 1,3-butanediol, 2,3-butanediol, diethylene glycol, triethylene glycol, tetraethylene glycol, polyethylene glycol with average molecular weight of 200, 300, 400, 600, 900, 1000, 1500 and 2000, dipropylene glycol, polyproylene glycol with average molecular weight of 425, 725, 1000, and 2000, 2-pyrrolidone, 1 methyl-2-pyrrolidone, 1-methyl-2-piperidone, N-ethylacetamide, N-methlpropionamide, N-acetyl ethanolamine, N-methylacetamide, formamide, 3-amino-1, 2-propanediol, 2,2-thiodiethanol, 3,3-thiodipropanol, tetramethylene sulfone, butadiene sulfone, ethylene carbonate, butyrolacetone, tetrahydrofurfuryl alcohol, glycerol, 1,2,4-butenetriol, trimethylpropane, pantothenol, Liponic EG-1. Preferred humectants are polyethylene glycol with average molecular weight of 400 to 1000, 2-pyrrolidone 2,2-thiodiethanol, and 1,5-pentanediol. Preferred penetrants include n-propanol, isopropyl alcohol, 1,3-propanediol, 1,2-hexanediol, and hexyl carbitol.

The amount of humectant used is determined by the properties of the ink and may range from 1-30%, preferably 5-15%, by weight, based on the total weight of the ink. Examples of commonly used humectants useful in forming the ink are: glycols, polyethylene glycols, glycerol, ethanolamine, diethanolamine, alcohols, and pyrrolidones. Other humectants known in the art may be used as well.

The use of suitable penetrants will depend on the specific application of the ink. Useful examples include pyrrolidone, and N-methyl-2-pyrrolidone. In a preferred embodiment in a inkjet ink composition containing more than 20%, on an equivalents basis, of hydroxy, amino, or thiol functionality relative to the hydroxy functionality of the emulsion polymer, the hydroxy, amino, or thiol functionality being present in, for example, penetrants, humectants, surfactants, etc., that such ingredients have a boiling point less than 220 °C, preferably less than 200 °C.

The amount of defoaming agent in the ink will typically range from 0-0.5% by weight, based on the total weight of the ink. Defoaming agents useful in forming aqueous dispersions of pigments are well known in the art and commercially available examples include Surfynol 104H and Surfynol DF-37 (Air Products, Allentown, PA).

In a preferred embodiment, particularly in those embodiments wherein methylol acrylamide or methylol methacrylamide is included in the copolymer, a catalyst which is a latent source of acidity, that is, a compound effective to lower the pH of the coating composition under the drying and curing conditions disclosed below, is included in the coating composition, preferably in an amount effective to provide a coating composition having a pH from about 1 to about 4.

The catalyst, which may be used at a level of 0 to 10 %, preferably 0.1% to 10%, more preferably 0.5% to 6%, most preferably 3% to 6%, by weight based on the weight of the ink composition includes, for example, ammonium chloride, ammonium nitrate, ammonium citrate, diammonium phosphate, magnesium chloride, amine salts of p-toluene sulfonic acid and mixtures thereof.

The ink compositions of the present invention may be prepared by any method known in the art for making such compositions, for example, by mixing, stirring or agitating the ingredients together using any art recognized technique to form an aqueous ink. The procedure for preparation of the ink composition of the present invention is not critical except to the extent that the ink composition is homogenous.

The ink composition of the present invention is applied by one of the inkjet techniques known in the art using, for example, thermal or bubble jet printers, piezoelectric printers, continuous flow printers, air brush or valve jet printers, to a substrate. Preferred substrates are fabrics, either woven or nonwoven, which may be formed from suitable fibers such as, for example, cotton, polyester, aramid, silk, acrylic, wool, rayon, nylon, polyamide, and glass. Any suitable substrate may be utilized, including paper, vinyl, leather and polyester. The ink composition is then cured, i.e., dried and crosslinked, at a selected time and temperature, times from 1 second to 10 minutes and temperatures from 60 °C to 300 °C being typical. It is understood that shorter cure times will ordinarily require higher temperatures to effect cure. The cure may be effected by combinations of thermal and radiation energy, such as microwave or infrared radiation.

Test Methods

Ink Jet Print Durability. Cured print samples are subjected to the accelerated 3A wash test of AATCC Test Method 61-1996. A rating of 5 means there is almost no color loss after wash and a rating of 1 means very significant color loss after wash.

The abbreviations listed below are used throughout the examples.

| | |
|---|---|
| MA | = Methyl acrylate |
| BA | = Butyl acrylate |
| EA | = Ethyl acrylate |
| AN | = Acrylonitrile |
| EHA | = 2-Ethylhexyl methacrylate |
| BMA | = Butyl methacrylate |
| IA | = Itaconic acid |
| MLAM | = N-methylolacrylamide |
| AM | = Acrylamide |
| DI water | = deionized water |
| TMPTA | = trimethylol propane triacrylate |
| MMA | = methylmethacrylate |
| AAEM | = 2-(Acetoacetoxy)ethyl methacrylate |
| DMAEMA | = Dimethylaminoethylmethacrylate |
| MAA | = Methacrylic acid |
| IPA | = Isopropylalcohol |

The following examples are illustrative of the invention.

**Example 1 (comparative): Aqueous PNP Dispersion**

A dispersion of butyl acrylate / methyl methacrylate / methacrylic acid /trimethylol propane triacrylate (37.5/37.5/15/10 wt. %) PNPs is prepared via solution polymerization in IPA as follows: A 5 liter reactor is fitted with a thermocouple, a temperature controller, a purge gas inlet, a water-cooled reflux condenser with purge gas outlet, a stirrer, and a monomer feed line. To a separate vessel is charged 450 grams of a monomer mixture (A) containing 169 g BA, 169 g MMA, 45 g TMPTA, 68 g MAA. To an additional vessel is charged an initiator mix (B) consisting of 18 g of a 75% solution of t-amyl peroxypivalate in mineral spirits (Triganox brand 125-C75), and 113 g isopropyl alcohol. A charge of 2330 g IPA is added to the reactor. After sweeping the reactor with nitrogen for approximately 30 minutes, heat is applied to bring the reactor charge to 79°C. When the contents of the reactor reaches 79°C, a dual feed of monomer mixture (A) and initiator mix (B) is fed uniformly using feed pumps over 120 minutes. At the end of the monomer and initiator feeds, the batch is held at 79°C for 30 minutes before adding the first of three initiator chasers consisting of 9 g of a 75% solution of t-amyl peroxypivalate in mineral spirits (Triganox brand 125-C75), and 22.5 g IPA. A second initiator chaser addition is made 30 minutes after the first initiator chaser addition. Similarly, a final initiator chaser addition is made 30 minutes after the second initiator chaser addition. The batch is then held at the polymerization temperature of 79°C for an additional 2½ hours to achieve full conversion of monomer. Solvent is removed in vacuo and an aqueous solution of dilute ammonium hydroxide is added. The resulting aqueous PNP dispersion can be used as a binder, such as for use in preparing ink jet inks.

**Example 2: Thermally Curable PNP Dispersion**

A dispersion of butyl acrylate / methyl methacrylate / methacrylic acid /trimethylol propane triacrylate / itaconic acid (37.5/37.5/5/10/10wt. %) PNPs is prepared via solution polymerization in IPA as follows: A 5 liter reactor is fitted with a thermocouple, a temperature controller, a purge gas inlet, a water-cooled reflux condenser with purge gas outlet, a stirrer, and a monomer feed line. To a separate vessel is charged 450 grams of a monomer mixture (A) containing 169 g BA, 169 g MMA, 45 g TMPTA, 23 g MAA, 45 g IA. To an additional vessel is charged an initiator mix (B) consisting of 18 g of a 75% solution of t-amyl peroxypivalate in mineral spirits (Triganox brand 125-C75), and 113 g isopropyl alcohol. A charge of 2330 g IPA is added to the reactor. After sweeping the reactor with nitrogen for approximately 30 minutes, heat is applied to bring the reactor charge to 79°C. When the contents of the reactor reaches 79°C, a dual feed of monomer mixture (A) and initiator mix (B) is fed uniformly using feed pumps over 120 minutes. At the end of the monomer and initiator feeds, the batch is held at 79°C for 30 minutes before adding the first of three initiator chasers consisting of 9 g of a 75% solution of t-amyl peroxypivalate in mineral spirits (Triganox brand 125-C75), and 22.5 g IPA. A second initiator chaser addition is made 30 minutes after the first initiator chaser addition. Similarly, a final initiator chaser addition is made 30 minutes after the second initiator chaser addition. The batch is then held at the polymerization temperature of 79°C for an additional 2½ hours to achieve full conversion of monomer. Solvent is removed in vacuo and an aqueous solution of dilute ammonium hydroxide is added. The resulting PNP dispersion can be used as a binder, such as for use in preparing ink jet inks.

**Example 3: Thermally Curable PNP Dispersion**

A dispersion of butyl acrylate / methyl methacrylate/ methylolacrylamide / methacrylic acid /trimethylol propane triacrylate (38/38/9/5/10 wt. %) PNPs is prepared via solution polymerization according to the method described in Example 1. The resulting aqueous PNP dispersion can be used as a binder, such as for use in ink jet inks.

**Example 4 (comparative): Standard Emulsion Polymer**

A dispersion of butyl acrylate / methyl methacrylate/ methacrylic acid (45/45/10 wt. %) is prepared via solution polymerization according to the method described in Example 1. The resulting aqueous emulsion can be used as a binder, such as for use in ink jet inks.

**Example 5: Thermally Curable PNP Dispersion**

A dispersion of butyl acrylate / methyl methacrylate / methacrylic acid /trimethylol propane triacrylate / itaconic acid (32.5/32.5/15/10/10wt. %) PNPs is prepared via solution polymerization according to the method described in Example 2. To 180 g of the resultant PNP is added 4.0 g glycerol and 1.4 grams of sodium hypophosphite monohydrate. The resulting aqueous emulsion can be used as a binder, such as for use in ink jet inks.

**Example 6: Thermally Curable PNP Dispersion**

A dispersion of butyl acrylate / methyl methacrylate / methacrylic acid /trimethylol propane triacrylate / 2-(actetoacetoxy) ethyl methacrylate (32.5/32.5/15/10/10wt. %) PNPs is prepared via solution polymerization according to the method described in Example 2. To the resultant dispersion is added 5% Ethox SAM-50, available from Ethox Chemicals, LLC in Greenville, SC, U.S.A. The resultant emulsion can be used as a binder, such as for use in ink jet inks.

**Example 7: Thermally Curable PNP Dispersion**

A dispersion of butyl acrylate / methyl methacrylate / methacrylic acid /trimethylol propane triacrylate / glycidyl methacrylate (32.5/32.5/15/10/10wt. %) PNPs is prepared via solution polymerization according to the method described in Example 2. To the resultant dispersion is added 5% Jeffamine brand T-3000, available from Huntsman Corporation, USA. The resultant emulsion can be used as a binder, such as for use in ink jet inks.

**Example 8: Thermally Curable PNP Dispersion**

A dispersion of butyl acrylate / methyl methacrylate / methacrylic acid /trimethylol propane triacrylate / glycidyl methacrylate (32.5/32.5/15/10/10wt. %) PNPs is prepared via solution polymerization according to the method described in Example 2. To the resultant dispersion is added 5% 3-Trimethoxysilylpropylamine. The resultant emulsion can be used as a binder, such as for use in ink jet inks.

**Example 9: Thermally Curable PNP Dispersion**

A dispersion of butyl acrylate / methyl methacrylate / methacrylic acid /trimethylol propane triacrylate / 2-(actetoacetoxy) ethyl methacrylate (32.5/32.5/15/10/10wt. %) PNPs is prepared via solution polymerization according to the method described in Example 2. To the resultant dispersion is added 4 wt% based on polymer solids of Desmodur® XP-7063 water dispersible polyisocyanate supplied by Bayer corporation and the dispersion is stirred. The reaction mixture is then neutralized to a pH of 7.0 with aqueous $NH_4OH$. The resultant emulsion can be used as a binder, such as for use in ink jet inks.

**Example 10: Thermally Curable PNP Dispersion**

A dispersion of butyl acrylate / methyl methacrylate / methacrylic acid /trimethylol propane triacrylate / 2-phosphoethyl (meth)acrylate (32.5/32.5/15/10/10wt. %) PNPs is prepared via solution polymerization according to the method described in Example 2. To the dispersion is added 8.0 wt% based on polymer solids of Ucarlnk RTM XL-20 available from Union Carbide Chemicals. The resultant emulsion can be used as a binder, such as for use in ink jet inks.

**Example 11: Thermally Curable PNP Dispersion**

A dispersion of butyl acrylate / methyl methacrylate / methacrylic acid /trimethylol propane triacrylate / methacryloxypropyl trimethoxy silane (36/36/15/10/3wt. %) PNPs is prepared via solution polymerization according to the method described in Example 2. The resultant emulsion can be used as a binder, such as for use in ink jet inks.

**Example 12: Thermally Curable PNP Dispersion**

A dispersion of butyl acrylate / methyl methacrylate / methacrylic acid /trimethylol propane triacrylate / methacryloxypropyl trimethoxy silane (37.5/37.5/15/10wt. %) PNPs is prepared via solution polymerization according to the method described in Example 2. To the resultant dispersion is added 5% 3-Trimethoxysilylpropylamine. The resultant emulsion can be used as a binder, such as for use in ink jet inks.

**Example 13: Thermally Curable PNP Dispersion**

A dispersion of butyl acrylate / methyl methacrylate / methacrylic acid /trimethylol propane triacrylate / 2-(actetoacetoxy) ethyl methacrylate (32.5/32.5/15/10/10wt. %) PNPs is prepared via solution polymerization according to the method described in Example 2. To the resultant dispersion is added 5% 3-Triethoxysilylpropylamine. The resultant emulsion can be used as a binder, such as for use in ink jet inks.

**Example 14: Thermally Curable PNP Dispersion**

To the acid functional PNP of example 1 is added 10 g Epocros K-2020E, an oxazoline crosslinker available from Esprix Technologies, USA. The mixture is mixed for 1 hour. The resultant PNP dispersion can be used as a binder, such as for use in preparing ink jet inks.

**Example 15: Thermally Curable PNP Dispersion**

A dispersion of ethylene glycol dicyclopentadienyl ether methacrylate/ butyl acrylate / methyl methacrylate / methacrylic acid /trimethylol propane triacrylate (20/20/35/15/10wt. %) PNPs is prepared via solution polymerization according to the method described in Example 2. The resultant dispersion can be used as a binder, such as for use in ink jet inks.

**Example 16: Ink Formulations**

The polymer dispersions described in examples 1 through 15 can be formulated into cyan ink jet inks according to the combination of ingredients as shown in the Table below. Fifteen individual inks are prepared, one for each polymer dispersion of examples 1-15 by combining, on a weight percentage basis:

Table 1:

| Fifteen Cyan Ink Jet Ink Formulations | |
| --- | --- |
| Composition | Amount (wt. %) |
| Cyan Pigment (20% such as AcryJet™ Cyan 157) | 17.50 |
| Latex or PNP Binder (15% ), one each from Examples 1-15 | 30.00 |
| Ammonium nitrate (25% in Water) | 3.00 |
| N-methylpyrrolidone | 6.50 |
| Liponic EG-7 | 1.00 |
| Dynol 604 | 0.50 |
| 1,3-propanediol | 10.20 |
| DI water | 31.30 |

AcryJet™ is a trademark of the Rohm and Haas Company, Philadelphia, PA, U.S.A. Liponic EG-7 is available from Lipo Chemicals, Inc. Dynol 604 is available from Air Products company, U.S.A.

Each ink jet ink sample shown in Table 1 is applied to a substrate. The applied ink is then cured under the application of thermal energy. The ink jet inks containing dispersions from Examples 2, 3 and 5-15 will demonstrate improvements in one or more of the following properties over ink jet inks containing dispersions from Examples 1 and 4 when coated onto a substrate: Durability, crock resistance, color retention, smear resistance, water resistance, optical density, image quality and lightfastness.

### Example 17: Radiation Curable PNP Dispersion

A dispersion of butyl acrylate / methyl methacrylate / methacrylic acid /trimethylol propane triacrylate / dimethylaminoethylmethacrylate (37.5/37.5/5/10/10wt. %) PNPs is prepared via solution polymerization in IPA as follows: A 5 liter reactor is fitted with a thermocouple, a temperature controller, a purge gas inlet, a water-cooled reflux condenser with purge gas outlet, a stirrer, and a monomer feed line. To a separate vessel is charged 450 grams of a monomer mixture (A) containing 169 g BA, 169 g MMA, 45 g TMPTA, 23 g MAA, 45 g DMAEMA. To an additional vessel is charged an initiator mix (B) consisting of 18 g of a 75% solution of t-amyl peroxypivalate in mineral spirits (Triganox 125-C75), and 113 g isopropyl alcohol. A charge of 2330 g IPA is added to the reactor. After sweeping the reactor with nitrogen for approximately 30 minutes, heat is applied to bring the reactor charge to 79°C. When the contents of the reactor reach 79°C, a dual feed of monomer mixture (A) and initiator mix (B) is fed uniformly using feed pumps over 120 minutes. At the end of the monomer and initiator feeds, the batch is held at 79°C for 30 minutes before adding the first of three initiator chasers consisting of 9 g of a 75% solution of t-amyl peroxypivalate in mineral spirits (Triganox brand 125-C75), and 22.5 gm. IPA. A second initiator chaser addition is made 30 minutes after the first initiator chaser addition. Similarly, a final initiator chaser addition is made 30 minutes after the second initiator chaser addition. The batch is then held at the polymerization temperature of 79°C for and additional 2½ hours to achieve full conversion of monomer. At the end of the final hold, 40 g of glycidylmethacrylate is added to the mixture, and held at 79°C for 2 hours. Solvent is removed in vacuo and an aqueous solution of dilute $NH_4OH$ is added. The resulting PNP dispersion can be used as a UV curable binder, such as for use in preparing ink jet inks.

### Example 18: Radiation Curable PNP Dispersion

To the photocurable PNP of Example 16 is added 10 g of CN-981 (urethane acrylate oligomer available from Sartomer Company, USA). The mixture is mixed for 1 hour. The resulting PNP dispersion can be used as a UV curable binder, such as for use in preparing ink jet inks.

### Example 19: Radiation Curable PNP Dispersion

A dispersion of furfuryl methacrylate/ butyl acrylate / methyl methacrylate / methacrylic acid /trimethylol propane triacrylate (20/20/35/15/10wt. %) PNPs is prepared via solution polymerization according to the method described in Example 2. The resultant dispersion can be used as a binder, such as for use in ink jet inks.

**Example 20: Ink Formulations**

The polymer dispersions described in examples 17-19 can be formulated into cyan ink jet inks according to the combination of ingredients as shown in the Table below. Three individual inks are prepared, one for each polymer dispersion of examples 17-19 by combining, on a weight percentage basis:

Table 2:

| Three Cyan Ink Jet Ink Formulations | |
| --- | --- |
| **Composition** | **Amount (wt. %)** |
| Cyan Pigment (20% such as AcryJet™ Cyan 157) | 17.50 |
| Latex or PNP Binder (15% ), one each from Examples 17-19 | 25.00 |
| Ammonium nitrate (25% in Water) | 3.00 |
| N-methylpyrrolidone | 6.50 |
| Liponic EG-7 | 1.00 |
| Dynol 604 | 0.50 |
| 1,3-propanediol | 10.20 |
| DI water | 31.30 |
| Esacure DP 250 (photoinitiator) | 5.0 |

AcryJet™ is a trademark of the Rohm and Haas Company, Philadelphia, PA, U.S.A. Liponic EG-7 is available from Lipo Chemicals, Inc. Dynol 604 is available from Air Products company, U.S.A. Esacure DP 250 is a water dispersible photoinitiator available from Lamberti S. p. A., Italy.

Each ink jet ink sample shown in Table 2 plus the ink samples made from the PNP dispersions of Examples 1 and 4 via the method of Example 16, is applied to a substrate. The applied ink is then cured under a UV lamp to initiate reaction of the photocurable functionality. The ink jet inks containing dispersions from Examples 17-19 will demonstrate improvements in one or more of the following properties over ink jet inks containing dispersions from Examples 1 and 4 when coated onto a substrate: Durability, crock resistance, color retention, smear resistance, water resistance, optical density, image quality and lightfastness.

**Claims**

1. A binder composition comprising polymeric nanoparticles ("PNPs") having a mean diameter in the range of 1 to 50 nanometers, the PNPs comprising as polymerized units 1-20%, by weight based on dry polymer weight, of one or more curable compositions which are unreactive at ambient conditions and are capable of being intiated thermally, chemically or via actinic radiation.

2. The binder composition of Claim 1 wherein one or more of the thermally curable compositions is selected from the group consisting of:

   (a) methylol acrylamide, methylol methacrylamide, methyl acrylamidoglycolate methyl ether, acrylamidoglycolic acid;
   (b) a polyacid comprising at least two carboxylic acid groups, anhydride groups, or salts thereof, wherein said carboxylic acid groups, anhydride groups, or salts thereof are neutralized to an extent of less than about 35% with a fixed base;
   (c) a polyol comprising at least two hydroxyl groups or hydroxylamine selected from the group consisting of diisopropanolamine, 2-(2-aminoethylamino)- ethanol, triethanolamine, tris(hydroxymethyl)aminomethane, and diethanolamine;
   (d) a copolymerized ethylenically-unsaturated monomer having a pendant group selected from the group consisting of acetoacetate, acetoacetamide, cyanoacetate, cyanoacetamide, an alkyl polyglycoside and glycidyl (meth)acrylate;
   (e) a covalently bonded compound having at least one unreacted isocyanate functional group;

(f) a copolymerized ethylenically-unsaturated monomer consisting of at least one sulfur- or phosphorous-containing acid group having an acid number of from 5 to 100, including 2-(meth)acrylamido-2-methyl-1-propanesulfonic acid, 3-sulfopropyl (meth)acrylate, 2-sulfoethyl (meth)acrylate, and 2-phosphoethyl (meth)acrylate;

(g) an aliphatic polycarbodiimide;

(h) a copolymerized ethylenically-unsaturated monomer having a pendant group capable of undergoing a Diels Alder reaction, including furfuryl, dicyclopentenyl, dicyclopentadienyl; and

(i) mixtures therof.

3. The binder composition of Claim 1 wherein one or more of the radiation curable compositions is glycidylmethacrylate, furfuryl methacrylate, dicyclopentenyl ether acrylate, dicyclopentadienyl ether methacrylate

4. An ink composition comprising the binder of Claim 1, a liquid medium and a colorant.

5. An ink composition comprising the binder of Claim 2, a liquid medium and a colorant.

6. An ink composition comprising the binder of Claim 3, a liquid medium and a colorant.

7. A method for improving the durability of inkjet ink printed on a substrate comprising:

(a) forming an inkjet ink composition comprising a liquid medium, a colorant and a binder composition comprising polymeric nanoparticles ("PNPs") having a mean diameter in the range of 1 to 50 nanometers, the PNPs comprising as polymerized units 1-20%, by weight based on dry polymer weight, of one or more curable compositions which are unreactive at ambient conditions and are capable of being intiated thermally, chemically or via actinic radiation;

(b) printing the ink on a substrate; and

(c) curing the ink by applying thermal or radiation energy.

8. The method of Claim 7 wherein the substrate is selected from the group of woven fabrics, nonwoven fabrics, paper, plastics, cotton, polyester, aramid, silk, acrylic, wool, rayon, nylon, vinyl, leather, polyamide and glass.